(19) Europäisches Patentamt / European Patent Office / Office européen des brevets

(11) **EP 2 711 431 B1**

(12) **EUROPEAN PATENT SPECIFICATION**

(45) Date of publication and mention
of the grant of the patent:
**14.12.2016 Bulletin 2016/50**

(51) Int Cl.:
***C12Q 1/68*** *(2006.01)*

(21) Application number: **12185698.3**

(22) Date of filing: **24.09.2012**

(54) **Method for determining the age of a human individual**

Verfahren zur Bestimmung des Alters einer menschlichen Einzelperson

Procédé pour déterminer l'âge d'un individu humain

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(43) Date of publication of application:
**26.03.2014 Bulletin 2014/13**

(73) Proprietor: **Rheinisch-Westfälische Technische Hochschule (RWTH) Aachen
52056 Aachen (DE)**

(72) Inventors:
  • **Wagner, Wolfgang**
    **52078 Aachen (DE)**
  • **Koch, Carmen**
    **52066 Aachen (DE)**
  • **Qiong, Lin**
    **52074 Aachen (DE)**

(74) Representative: **Remus, Alvaro Johannes
BPSH Schrooten Haber Remus
Patent- und Rechtsanwaltspartnerschaft mbB
Mörsenbroicher Weg 191
40470 Düsseldorf (DE)**

(56) References cited:
  • **KOCH CARMEN M ET AL: "Epigenetic-aging-signature to determine age in different tissues", AGING-US, vol. 3, no. 10, October 2011 (2011-10), pages 1018-1027, XP002687316, ISSN: 1945-4589**
  • **BOCKLANDT SVEN ET AL: "Epigenetic Predictor of Age", PLOS ONE, vol. 6, no. 6, June 2011 (2011-06), XP002687317, ISSN: 1932-6203**
  • **BOLLATI V ET AL: "Decline in genomic DNA methylation through aging in a cohort of elderly subjects", MECHANISMS OF AGEING AND DEVELOPMENT, ELSEVIER SEQUOIA, LAUSANNE, CH, vol. 130, no. 4, 1 April 2009 (2009-04-01) , pages 234-239, XP025988022, ISSN: 0047-6374, DOI: 10.1016/J.MAD.2008.12.003 [retrieved on 2008-12-27]**
  • **SIMONE BORK ET AL: "DNA methylation pattern changes upon long-term culture and aging of human mesenchymal stromal cells", AGING CELL, BLACKWELL PUBLISHING, GB, vol. 9, no. 1, 1 February 2010 (2010-02-01), pages 54-63, XP002663876, ISSN: 1474-9718, DOI: 10.1111/J.1474-9726.2009.00535.X [retrieved on 2009-11-06]**
  • **SOMMER ANKE ET AL: "Expression of aspartoacylase (ASPA) and Canavan disease", GENE, vol. 505, no. 2 , pages 206-210, XP028930448, ISSN: 0378-1119, DOI: 10.1016/J.GENE.2012.06.036**

**EP 2 711 431 B1**

## Description

<u>Background of the invention</u>

The invention relates to a method for determining the age of a human donor, preferably the biological age of a human donor.

<u>Prior art</u>

All tissues of the organism are affected by aging. Aging has different consequences in different tissues - it results for example in wrinkle formation of dermis, graying of epidermally-derived hair, loss of bone formation, myeloid bias of blood, and compromised function of the immune system. Despite this wide spectrum of tissue specific age-associated changes the underlying molecular mechanisms might be related. Aging has been associated with accumulation of cellular defects such as DNA damage and telomere shortening. On the other hand, there is accumulating evidence that aging rather resembles a developmentally regulated process which is tightly controlled by specific epigenetic modifications (Schellenberg et al., 2011; Koch et al., 2011; Gonzalo, 2010; Murgatroyd et al., 2010; Marciniak-Czochra et al., 2009). An epigenetic trait is a stably heritable phenotype resulting from changes in a chromosome without alterations in the DNA sequence (Berger et al., 2009). Among epigenetic modifications, DNA methylation is best characterized. CpG-dinucleotides in the mammalian genome can be enzymatically methylated at specific cytosine residues in the DNA (CpG sites) and many studies demonstrated the occurrence of age-associated modifications in the DNA methylation pattern (Christensen et al., 2009; Maegawa et al., 2010).

Koch and Wagner (2011) have identified an Epigenetic-Aging-Signature which is applicable for many tissues to predict donor age. DNA-methylation profiles of various cell types were retrieved from public data depositories - all using the HumanMethylation27 BeadChip platform (Illumina, Inc., San Diego, USA) which represents 27,578 CpG sites. Five datasets from dermis, epidermis, cervical smear, T-cells and monocytes were used for Pavlidis Template Matching to identify 19 CpG sites that are continuously hypermethylated upon aging (R > 0.6; p-value <$10^{-13}$). Four of these CpG sites (associated with the genes NPTX2, TRIM58, GRIA2 and KCNQ1 DN) and an additional hypomethylated CpG site (associated with the gene BIRC4BP) were implemented in a model to predict donor age. This Epigenetic-Aging-Signature was tested on a validation group of eight independent datasets corresponding to several cell types from different tissues. Overall, the five CpG sites revealed age-associated DNA-methylation changes in all tissues. The average absolute difference between predicted and real chronological age was about 11 years.

Bocklandt et al. (2011) identified 88 sites in or near 80 genes, including ASPA, PDE4C and KCNK12, for which the degree of methylation is correlated with age in saliva. Only some one of them, e.g. KCNK12, were found to be positively correlated with age in blood samples.

However, to date there is no reliable and precise method for determining the biological age on a molecular level using blood samples of a donor.

<u>Summary of the invention</u>

It is the object of the invention to provide a method and tools for determining the age of human individuals from blood samples of a donor, in particular the biological age of said donor.

The object is achieved by providing a method for determining the age of a human donor, preferably the biological age of a human donor, comprising:

- Providing a blood sample including blood cells of said donor;
- Determining methylation levels of at least two CpG-dinucleotides of at least two specific regions of at least one chromosome of said blood cells, wherein one of said specific regions is the ASPA gene and at least one further specific region is selected from the group consisting of RAB36, ITGA2B, GBP1, PDE4C, KCNK12, and CBX7; and
- Determining the age of said donor by comparing said determined methylation levels with empirically determined data representing a correlation between the methylation levels of said CpG-dinucleotides and the chronological age of at least one human individual.

Advantageously, the methylation level of the CpG-dinucleotides of the ASPA (aspartoacylase) gene correlates with the age of the donor of the blood cells, i.e. the CpG-dinucleotides are continuously demethylated with increasing age, wherein the blood cells may be provided in the form of whole blood so that the age of the donor can be determined directly from a blood sample of this donor fast and straightforward. Surprisingly, this correlation is observed in all blood cell types so that the age of the donor can be determined directly from blood samples by comparing the determined methylation level with empirically determined data. The comparison may be accomplished, for example, by regression analysis or any other suitable statistical method, preferably by linear regression. Preferably, the CpG-dinucleotide is the dinucleotide cg02228185 of the ASPA gene (the foregoing number refers to HumanMethylation27 BeadChip of Illumina, Inc., San Diego, USA).

According to the invention, additionally a methylation level of at least one further CpG-dinucleotide of at least one further specific region of at least one chromosome of said blood cells is determined, wherein the further specific region of the chromosome is at least one gene selected from the group consisting of RAB36 (RAB36; member RAS oncogene family), ITGA2B (integrin alpha 2b precursor), GBP1 (guanylate binding protein 1; interferon-inducible; 67kD), PDE4C (phosphodiesterase 4C; cAMP-specific - phosphodiesterase E1 dunce homolog; Drosophila), KCNK12 (potassium channel; subfamily K; member 12), and CBX7 (chromobox homolog 7). Preferably, the further CpG-dinucleotide is at least one dinucleotide selected from the group consisting of cg15379633 of the RAB36 gene, cg25809905 of the ITGA2B gene, cg13406950 of the GBP1 gene, cg17861230 of the PDE4C gene, cg27320127 of the KCNK12 gene, and cg23124451 of the CBX7 gene (foregoing numbers refer to HumanMethylation27 BeadChip of Illumina, Inc., San Diego, USA).

Nucleotide sequence (DNA, plus strand) of the ASPA gene on Illumina 27k BeadChip (CpG-dinucleotide at position 61/62 (cg02228185) is underlined and printed in bold letters):

TCACTGCTCTGGGGTTAGTAATAAATGGTTTTACCTCCAGCCCTGTTCTCTGA ATCTCAG**CG**CCATTCTCTAGCCAATGCTTAACCAGAAATACTCCGGTTAGCTC ATTCCCATGGGTTCCT (SEQ ID NO: 1)

Nucleotide sequence (DNA, plus strand) of the RAB36 gene on Illumina 27k BeadChip (CpG-dinucleotide at position 61/62 (cg15379633) is underlined and printed in bold letters):

CCAGACTCCAGGCAGGTTCTCGTTGCTATGGTGATCGCCGGTGCAAGCTGG ATGCTTGGA**CG**CGCCGCTGCCAGTCCAACGCAGACCCCGCCCACGACGTCG ACGATTCGTGTAGCCCGCAG (SEQ ID NO:2)

Nucleotide sequence (DNA, minus strand) of the ITGA2B gene on Illumina 27k BeadChip (CpG-dinucleotide at position 61/62 (cg25809905) is underlined and printed in bold letters):

CCTGTGAACGGACCAAGAGTAAACAGTGTGCTCAATGCTGTGCCTACGTGTG TTAGCCCA**CG**CGGCCAGCCTGAGGAGTCAGGGAAGGCTCCCCTAGGCAAAG CCCCCAACCAGAATCAAGT (SEQ ID NO: 3)

Nucleotide sequence (DNA, minus strand) of the GBP1 gene on Illumina 27k BeadChip (CpG-dinucleotide at position 61/62 (cg13406950) is underlined and printed in bold letters):

AGTTCATTTTCTTAAACATCATCAGAACCAAATTAGGCTGCAGATGAGCCTGA AGTGGGA**CG**CAGGTCAGATGAAATCCTGGTGTTATCAGGGACAGCATGGCC TTAAGTGCCACTACAGTG (SEQ ID NO: 4)

Nucleotide sequence (DNA, minus strand) of the PDE4C gene on Illumina 27k BeadChip (CpG-dinucleotide at position 61/62 (cg17861230) is underlined and printed in bold letters):

GCGGACTTGTCCGGATCCGAATAGAAGCGCTGTTGGATGCGGATGGGGCGC CGGGGTTGC**CG**CCACAGGTGCTTCGGGGCTCTGGTCATGCTGTGGCGGCC GCGAGAGCGACTCAACCTGCT (SEQ ID NO: 5)

Nucleotide sequence (DNA, minus strand) of the KCNK12 gene on Illumina 27k BeadChip (CpG-dinucleotide at position 61/62 (cg27320127) is underlined and printed in bold letters):

ACGAAGGGAACTTAAACTTCGGACTAACGCCCGAGAATCCAGTGTCGGCGAT TCGCGTCG**CG**CGGCCTCCAGCCCTGGAGACCTCAGAGCACCACCAACTTGA GCAAAGTTTCACCGCGGCA (SEQ ID NO: 6)

Nucleotide sequence (DNA, minus strand) of the CBX7 gene on Illumina 27k BeadChip (CpG-dinucleotide at position 61/62 (cg23124451) is underlined and printed in bold letters):

TCAGTCTCCCCATATTTACAATAAAAGGGGAGCGAGGTGGGATGGCGCTGAG GATCCCTA**CG**TCCGATCCTAATCTCCAGCTCAGGCAGGCTCGGCCGCCACTA GCATCCTGGAGCGACAAC (SEQ ID NO: 7)

According to a further advantageous aspect of the invention one of the specific regions of the chromosome comprises the nucleotide sequence of SEQ ID NO: 1. Preferably, one of the at least two CpG-dinucleotides is a dinucleotide consisting of nucleotides no. 61 and 62 (61/62) of the nucleotide sequence of SEQ ID NO: 1. The methylation level of the CpG-dinucleotide at position 61/62 linearly depends on the chronological age of the human individual so that this CpG site is suitable for determining the age from blood samples of a human donor. This CpG-dinucleotide is continuously demethylated with increasing age of the donor, i.e. a straight proportional decrease of the methylation level of this CpG-dinucleotide can be observed in different blood cell types. Advantageously, the correlation between the age of a donor and the methylation level of the above-identified CpG-dinucleotide is observed in all blood cell types so that the age of the donor can be determined directly from blood samples.

According to a further advantageous aspect of the invention the at least one further specific region of the chromosome comprises at least one nucleotide sequence selected from the group consisting of SEQ ID NO: 2, SEQ ID NO: 3, SEQ ID NO: 4, SEQ ID NO: 5, SEQ ID NO: 6, and SEQ ID NO: 7. Preferably, the additional CpG-dinucleotide of said at least one further specific region is at least one dinucleotide consisting of nucleotides no. 61 and 62 (61/62) of one of the nucleotide sequences selected from the group consisting of SEQ ID NO: 2, SEQ ID NO: 3, SEQ ID NO: 4, SEQ ID NO: 5, SEQ ID NO: 6, and SEQ ID NO: 7. The methylation level of the additional CpG-dinucleotides at position 61/62 linearly depends on the chronological age of the human individual so that these CpG sites are suitable for determining the age from blood samples of a human donor. These additional CpG-dinucleotides are continuously methylated or demethylated with increasing age of the donor, i.e. a straight proportional increase or decrease of the methylation level of these CpG-dinucleotides can be observed in different blood cell types.

According to another advantageous embodiment of the invention one of the at least two CpG-dinucleotides is at least one of the CpG-dinucleotides within a region of about 50,000 nucleotides upstream and/or downstream, preferably of about 40,000 nucleotides upstream and/or downstream, more preferably of about 30,000 nucleotides upstream and/or downstream, even more preferably of about 20,000 nucleotides upstream and/or downstream, even more preferably of about 10,000 nucleotides upstream and/or downstream, even more preferably of about 5,000 nucleotides upstream and/or downstream, even more preferably of about 1,000 nucleotides upstream and/or downstream of the CpG-dinucleotide consisting of nucleotides no. 61 and 62 of the nucleotide sequence of SEQ ID NO: 1. The CpG-dinucleotides within the above-identified regions upstream and downstream of the specific CpG-dinucleotide described above are also well suited to determine the age of the donor using the method according to the invention. With these CpG-dinucleotides a straight proportional change of the methylation level of these CpG-dinucleotides depending on the age of the donor can be observed as well. That is, they are also continuously demethylated with increasing age. For example, the CpG-dinucleotide at position 97/98 of SEQ ID NO: 1 may also be used for determining the age of a human donor from his/her blood cells. Advantageously, the correlation between the age of a donor and the methylation level of the above-identified CpG-dinucleotide is observed in all blood cell types so that the age of the donor can be determined directly from blood samples.

According to another advantageous embodiment of the invention the additional CpG-dinucleotide of the at least one further specific region is at least one of the CpG-dinucleotides within a region of about 50,000 nucleotides upstream and/or downstream, preferably of about 40,000 nucleotides upstream and/or downstream, more preferably of about 30,000 nucleotides upstream and/or downstream, even more preferably of about 20,000 nucleotides upstream and/or downstream, even more preferably of about 10,000 nucleotides upstream and/or downstream, even more preferably of about 5,000 nucleotides upstream and/or downstream, even more preferably of about 1,000 nucleotides upstream and/or downstream of at least one CpG-dinucleotide consisting of nucleotides no. 61 and 62 of one of the nucleotide sequences selected from the group consisting of SEQ ID NO: 2, SEQ ID NO: 3, SEQ ID NO: 4, SEQ ID NO: 5, SEQ ID NO: 6, and SEQ ID NO: 7. The additional CpG-dinucleotides within the above-identified regions upstream and downstream of the specific CpG-dinucleotide(s) described above are also well suited to determine the age of the donor using the method according to the invention. With these additional CpG-dinucleotides a straight proportional change of the methylation level of these CpG-dinucleotides depending on the age of the donor can be observed as well. That is, they are also continuously methylated or demethylated with increasing age. For example, the CpG-dinucleotides at positions 21/22, 36/37, 39/40, 63/64, 66/67, 80/81, 89/90, 95/96, 98/99, 101/102, 104/105, 109/110, and 118/119 of SEQ ID NO: 2, positions 9/10, 47/48, and 63/64 of SEQ ID NO: 3, positions 2/3, 12/13, 18/19, 28/29, 40/41, 49/50, 52/53, 75/76, 97/98, 101/102, 103/1-04, and 109/110 of SEQ ID NO: 5, positions 2/3, 20/21, 28/29, 32/33, 46/47, 49/50, 54/55, 56/57, 59/60, 63/64, 116/117, and 118/119 of SEQ ID NO: 6, and positions 33/34, 46/47, 65/66, 93/94, 97/98 and 116/117 of SEQ ID NO: 7 may additionally be used for determining the age of a human donor from his/her blood cells.

Preferably, the blood cells are cells selected from the group consisting of monocytes, T cells, hematopoietic progenitor cells (HPCs), lymphocytes, mononuclear cells (MNCs), and leucocytes.

In a preferred embodiment of the invention, the data comprise at least one linear regression equation. Advantageously, the methylation level of the CpG-dinucleotides according to the invention linearly correlates with the age of the donor. The CpG-dinucleotides are continuously methylated or demethylated with increasing age, i.e. a straight proportional change of the methylation level of these CpG-dinucleotides can be observed. That is, comparing the determined methylation level with empirically determined data may be accomplished in that the methylation level as determined is inserted into a formula for linear regression. Thereby, it is possible to determine the age of the donor by means of blood samples, even if no positive or negative control is available. Preferably, one equation comprises at least two linear regressions. This multivariate model combines two or more linear regressions into one equation. Instead of averaging multiple independent regression analyses, this single equation method is much easier to use.

One further aspect of the invention is the use of an artificial nucleic acid molecule for determining the age of a human donor according to the method as described above, wherein said artificial nucleic acid molecule comprising at least one nucleotide sequence selected from the group consisting of:

a) a nucleotide sequence comprising at least one sequence selected from the group consisting of SEQ ID NO: 8 to SEQ ID NO: 10;
b) a nucleotide sequence comprising at least one sequence selected from the group consisting of SEQ ID NO: 11 to SEQ ID NO: 13;
c) a nucleotide sequence comprising at least one sequence selected from the group consisting of SEQ ID NO: 14 to SEQ ID NO: 16;
d) a nucleotide sequence which has at least 90% identity, preferably at least 95% identity, with the nucleotide sequence of a), b) or c);
c) a nucleotide sequence which corresponds to the complementary strand of the nucleotide sequence of a), b), c) or d).

One further aspect of the invention is the use of a nucleic acid molecule for determining the age of a human donor, preferably the biological age of a human donor, according to the method as described above, wherein said nucleic acid molecule comprises at least one nucleotide sequence selected from the group consisting of:

a) a nucleotide sequence comprising at least one sequence selected from the group consisting of SEQ ID NO: 1, SEQ ID NO: 2, SEQ ID NO: 3, SEQ ID NO: 4, SEQ ID NO: 5, SEQ ID NO: 6, and SEQ ID NO: 7;
b) a nucleotide sequence which differs from the nucleotide sequence of a) by replacement of at most 10 % of the nucleotides, preferably at most 5 % of the nucleotides, but for said CpG-dinucleotide; and
c) a nucleotide sequence which corresponds to the complementary strand of the nucleotide sequence of a) or b).

The methylation level can be determined, for example, by methylation specific PCR, sequence analysis of bisulfite-treated DNA, CHIP-sequencing (Illumina Human Methylation BeadChip Technology), Methyl-CAP-sequencing, Next-Generation-Sequencing, COBRA-Assay and methylation-specific restriction patterns. Alternatively, it is possible to determine the methylation level by MassARRAY assay. It is preferred that the methylation status is determined by pyrosequencing of bisulfite-treated DNA as described in more detail herein above, and which allows identifying whether a specific CpG-dinucleotide was methylated or not, and thereby provides an accurate value for the percentage of methylated CpG-dinucleotides of a given CpG-dinucleotide.

Determining the methylation level of specific CpG-dinucleotides is not only a suitable tool for determining the chronological (biographical) age of an individual but also allows conclusions on the biological age of this individual. In fact, a strong correlation between the methylation level of the specific CpG-dinucleotides according to the invention and the biological age of the donor can be observed. Analysis of biological aging is very helpful for optimized personalized medicine. In geriatrics, measure of fitness and frailty in elderly people is very important and daily routine. Parameters such as physical exercise, comorbidities and living conditions are for example used to support the decision between curative or palliative therapeutic approaches. This clinical assessment of "biological age" is so far not based on molecular parameters. The method according to the invention provides a quantitative and less susceptible measure than observation by the clinician. Furthermore, the quantitative molecular parameters provided by the invention assist further demarcation of relevant clinical parameters with impact on biological aging (e.g. body mass index, blood pressure, specific co-morbidities).

The invention is further described in detail with reference to the figures.

Brief description of the figures

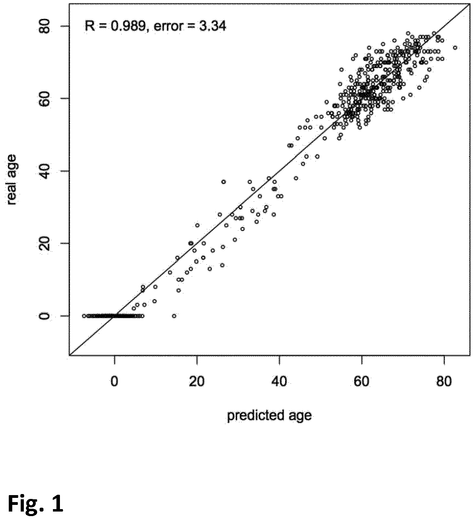

**Figure 1** shows a linear regression diagram representing the correlation between the chronological ("real") age (y-axis) and the predicted age (x-axis) after determination of the methylation level of 102 selected age-associated CpG-dinucleotides. Circles and dots respectively represent one methylation level value.

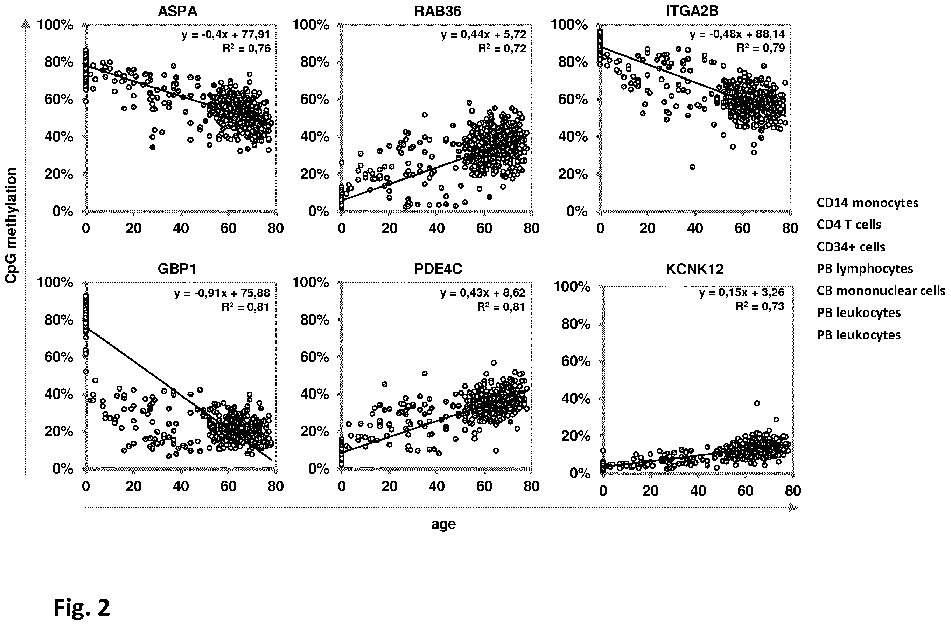

**Figure 2** shows a diagram representing the methylation level of six specific CpG-dinucleotides (position 61/62 of SEQ ID NO: 1 (ASPA), SEQ ID NO: 2 (RAB36), SEQ ID NO: 3 (ITGA2B), SEQ ID NO: 4 (GBP1), SEQ ID NO: 5 (PDE4C), and SEQ ID NO: 6 (KCNK12)) as a function of age. Analyses using Pavlidis Template Matching demonstrate significant changes of the methylation level in different blood cell types (CD14 monocytes, CD4 T cells, CD34 hematopoietic progenitor cells (HPC, CD34+ cells), peripheral blood (PB) lymphocytes, cord blood (CB) mononuclear cells (MNC), PB whole blood, and PB leucocytes). Circles and dots respectively represent one methylation level value of one cell type.

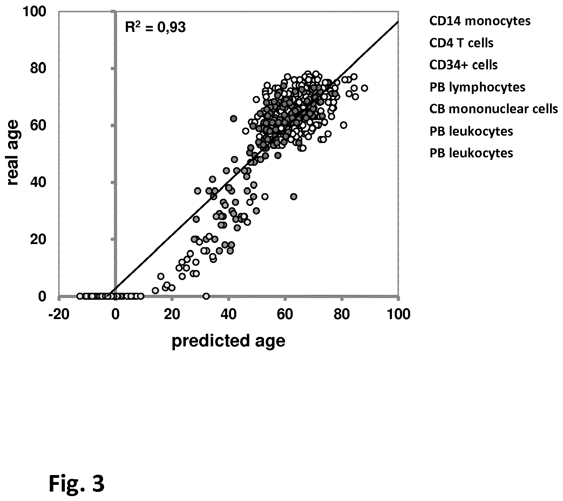

**Figure 3** shows a linear regression diagram representing the correlation between the chronological ("real") age (y-axis) and the predicted age (x-axis) based on the analyses of the six specific CpG-dinucleotides (position 61/62 of SEQ ID NO: 1 (ASPA), SEQ ID NO: 2 (RAB36), SEQ ID NO: 3 (ITGA2B), SEQ ID NO: 4 (GBP1), SEQ ID NO: 5 (PDE4C), and SEQ ID NO: 6 (KCNK12)) according to Figure 2.

Circles and dots respectively represent one methylation level value of one cell type.

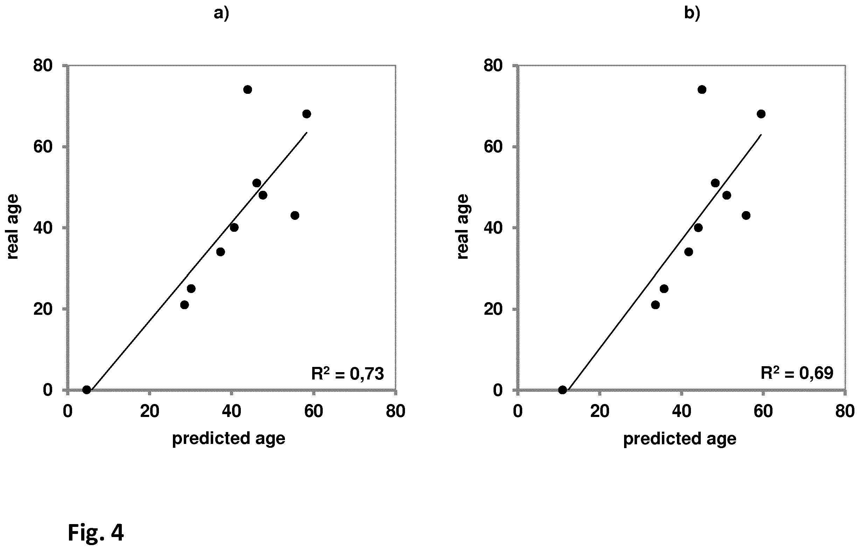

**Figure 4** shows linear regression diagrams representing the correlation between the chronological ("real") age (y-axis) and the predicted age (x-axis) after determination of the methylation level of three specific CpG-dinucleotides (ASPA: position 61/62 of SEQ ID NO: 1, ITGA2B: position 61/62 of SEQ ID NO: 3, PDE4C: position 61/62 of SEQ ID NO: 5) by pyrosequencing. Each dot represents one independent blood sample.

   a) Single linear regression model, average deviation of age: 7.84 years
   b) Multivariate model, average deviation of age: 10.30 years

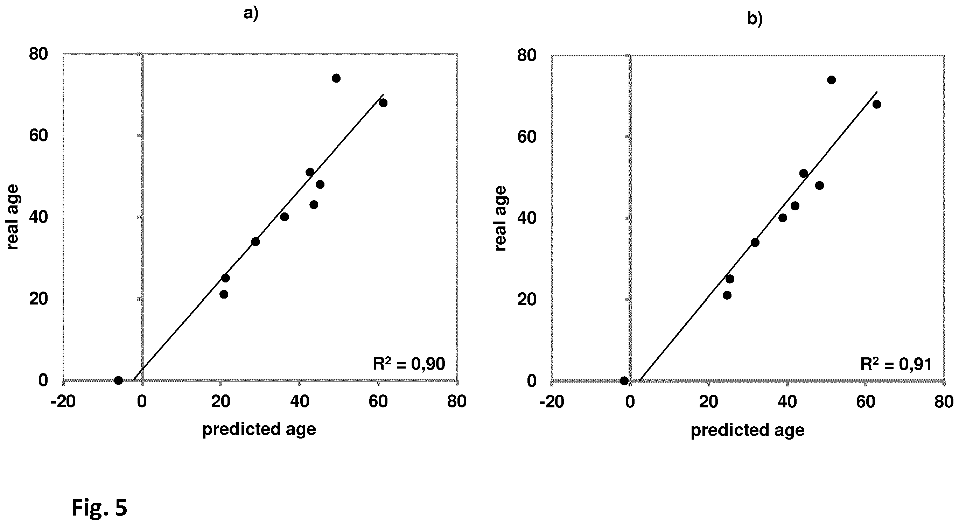

**Figure 5** shows linear regression diagrams representing the correlation between the chronological ("real") age (y-axis) and the predicted age (x-axis) after determination of the methylation level of three specific CpG-dinucleotides (ASPA: position 61/62 of SEQ ID NO: 1, ITGA2B: position 61/62 of SEQ ID NO: 3, PDE4C: position 75/76 of SEQ ID NO: 5) by pyrosequencing. Each dot represents one independent blood sample.

   a) Single linear regression model, average deviation of age: 6.25 years
   b) Multivariate model, average deviation of age: 4.53 years

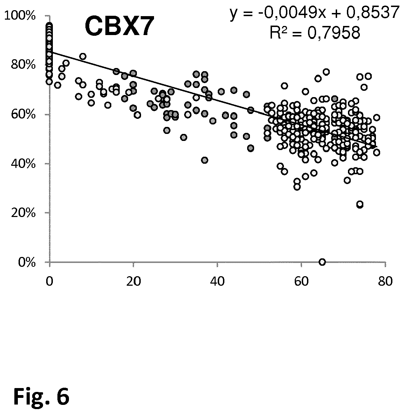

**Figure 6** shows a diagram representing the methylation level of a CBX7-associated CpG-dinucleotide (position 61/62 of SEQ ID NO: 7) as a function of age. Each circle (dot) represents one methylation level value.

Description of exemplary and preferred embodiments of the invention

Infinium Methylation Assays (Illumina, Inc., San Diego, USA) are well-suited to quantitatively interrogate methylation sites at single-nucleotide resolution. For example, the HumanMethylation27 BeadChip allows interrogation of 27,578 CpG loci, covering more than 14,000 genes. Alternatively, the HumanMethylation450 BeadChip offers a unique combination of comprehensive, expert-selected coverage of >450,000 methylation sites, high throughput, and low price, making it ideal for screening genome-wide association study (GWAS) populations.

Data of 598 samples from DNA-methylation arrays (illumina 27k BeadChip) of 5 studies (only blood samples) from NCBI GEO database were collected. To obtain the age-associated CpG sites, we calculated the correlation coefficient (R) between the chronological age of the samples and corresponding beta values for each CpG site (Pavlidis Template Matching). After pre-filtering, 102 age-associated CpG sites (R > 0.85 or R < -0.85) were retained for feature (CpG sites) selection. A multivariable linear model was trained using the complete feature set of 102 CpG sites. Leave one out cross-validation was applied for estimating the model performance. As a result, the average prediction error of the model was 3.34 years **(Figure 1)**.

In order to reduce the feature set size, the complete sample set was divided into training set and test set in terms of the chosen split-ratio (such that 50%:50%, 60%:40%, 70%:30%, 80%:20, 90%:10%). Next recursive feature elimination (RFE) was applied using a linear model on the training set. The feature size was limited up to six specific CpG sites (ASPA, RAB36, ITGA2B, GBP1, PDE4C, and KCNK12). **Figure 2** shows that the six specific CpG-dinucleotides at position 61/62 of SEQ ID NO: 1 (ASPA), SEQ ID NO: 2 (RAB36), SEQ ID NO: 3 (ITGA2B), SEQ ID NO: 4 (GBP1), SEQ ID NO: 5 (PDE4C), and SEQ ID NO: 6 (KCNK12) are continuously methylated (RAB36, PDE4C, KCNK12) or demethylated (ASPA, ITGA2B, GBP1) with increasing age. Surprisingly, this correlation is observed in all blood cell types so that the age of the donor can be determined directly from blood samples. It is demonstrated that there is a straight proportional change of the methylation level of these CpG-dinucleotides. Comparing the determined methylation level of the six sites with empirically determined data is accomplished in that the methylation level as determined is inserted into a formula for linear regression. Thereby, it is possible to predict the age of the donor, even if no positive or negative control is available. The prediction error with these six CpG sites was 6 years **(Figure 3).** In this example, one equation comprises six linear regressions. This multivariate model combines six linear regressions into one equation, instead of averaging six independent regression analyses.

**Figure 4** shows linear regression diagrams representing the correlation between the chronological age ("real age") and the predicted age after determination of the methylation level of three specific CpG-dinucleotides (ASPA: position 61/62 of SEQ ID NO: 1, ITGA2B: position 61/62 of SEQ ID NO: 3, PDE4C: position 61/62 of SEQ ID NO: 5). Ten blood samples of donors with known chronological age were analyzed. DNA was isolated, bisulfite converted and used for pyrosequencing. Based on the measurements of the three CpG sites it was possible to predict the chronological age with a mean precision of 7.84 years (a). This analysis clearly demonstrates that prediction of chronological age based on only three CpG sites is feasible in independent samples. Deviation at higher ages is probably due to differences in biological age. While a) shows a diagram based on the average methylation value of three independent linear regressions, b) shows a diagram based on the combination of the three linear regressions into one linear regression equation.

Predicted age = 96,59 - cg2228185*56,76 - cg2580995*64,23 + cg17861230*90,51, where the methylation beta value is inserted for the indicated CpG sites.

**Figure 5** shows linear regression diagrams representing the correlation between the chronological age ("real age") and the predicted age after determination of the methylation level of three specific CpG-dinucleotides as shown in Figure 4, wherein the methylation level of a different PDE4C-associated CpG site was analysed (ASPA: position 61/62 of SEQ ID NO: 1, ITGA2B: position 61/62 of SEQ ID NO: 3, PDE4C: position 75/76 of SEQ ID NO: 5). Diagram a) shows a linear regression analysis based on the average methylation value of three independent linear regressions. Diagram b) shows a linear regression analysis based on the combination of the three linear regressions into one linear regression equation (multivariate model). Ten blood samples of donors with known chronological age were analyzed. DNA was isolated, bisulfite converted and used for pyrosequencing. Based on the measurements of these three CpG sites it was possible to predict the chronological age with a mean precision of 6.25 years (a), which is even more precise than the result shown in Figure 4. Accordingly, it becomes apparent from this analysis that different CpG-dinucleotides within a specific region may be used as well for determining the age of a donor. Moreover, the mean precision of the analysis based on the multivariate model (b) is 4.43 years, which represents a very precise determination of donor's age.

**Figure 6** shows a diagram representing the methylation level of the CpG-dinucleotide at position 61/62 of SEQ ID NO: 7 (CBX7) as a function of age. As becomes apparent from this diagram, the CBX7-associated CpG-dinucleotide is continuously demethylated with increasing age, i.e. there is a straight proportional correlation of the methylation level of this CpG-dinucleotide with donor's age. Consequently, the CpG-dinucleotide at position 61/62 of SEQ ID NO: 7 (CBX7) is also suitable to predict the biological age of a donor.

Example

DNA isolation and bisulfite conversion:

Genomic DNA was isolated from blood cells using the QIAGEN DNA Blood Midi-Kit. DNA quality was assessed with a NanoDrop ND-1000 spectrometer (NanoDrop Technologies, Wilmington, USA) and gel electrophoresis. 600 ng DNA were subsequently bisulfite converted using the EpiTect Bisulfite Kit (Qiagen, Hilden, Germany).

DNA methylation profiling:

DNA methylation profiles were analyzed using the HumanMethylation27 or HumanMethylation450 Bead Chip (illumina, San Diego, USA) according to the manufacturer's instructions. During hybridization, the DNA molecules anneal to two different bead types with locus-specific DNA oligomers - one corresponds to the methylated (C) and the other to the unmethylated (T) state. Allele-specific primer annealing is followed by single-base extension using DNP- and Biotin-labeled ddNTPs. After extension, the array is fluorescently stained, scanned, and the intensities of the unmethylated and methylated bead types measured. Hybridization and initial data analysis with the BeadStudio Methylation Module were performed at the DKFZ Gene Core Facility in Heidelberg.

Analysis of DNA-methylation profiles:

Raw data of new datasets and recently published datasets were quantile normalized to minimize chip effects. Principal components analysis (PCA) was calculated with prcomp in R package stats. For selection of relevant CpG sites we used Pavlidis Template Matching performed with the MultiExperiment Viewer (MeV, TM4.6). Templates were specified that corresponded to the chronological age. The dataset was then searched for matches to the template, based on the Pearson Correlation between the template and methylation level values of the data set. For subsequent analysis we have only considered CpG sites with highly significant hyper- or hypo-methylation according to the corresponding templates ($P < 10^{-11}$). Based on this analysis, we have selected six CpG sites - for simplicity they were termed by their corresponding genes: ASPA, RAB36, ITGA2B, GBP1, PDE4C, and KCNK12. Methylation levels of these CpG sites were plotted against age for linear regression analysis with EXCEL 2007 (Microsoft). Based on these linear regressions we calculated the age ($N$) for each of the six CpG sites ($i$) by inserting the specific DNA-methylation levels for each gene ($\beta$).

$$N_i = (\beta_i - A_i)/B_i$$

where $A$ is the Y-axis intercept and $B$ is the slope of the corresponding CpG site in the training group. Subsequently, we determined the mean and standard deviation of the predictions of the six individual CpG sites as measure of age.

Pyrosequencing:

Independent blood samples of known donor's age were subsequently bisulfite converted and analyzed by pyrosequencing with regard to three of the six specific CpG sites (ASPA: position 61/62 of SEQ ID NO: 1, ITGA2B: position 61/62 of SEQ ID NO: 3, PDE4C: position 61/62 of SEQ ID NO: 5). Pyrosequencing was performed at Varionostic GmbH (Ulm, Germany). Primers and sequencing primers are provided in **Table 1.** Other primers than those disclosed herein may be used for amplifying and/or sequencing the respective nucleic acids.

The results of the analysis are briefly summarized in **Figure 4.** Figure 4 illustrates that there is a clear and unequivocal correlation of the age of a donor and the methylation level of each one of the three CpG-dinucleotides (ASPA: position 61/62 of SEQ ID NO: 1, ITGA2B: position 61/62 of SEQ ID NO: 3, PDE4C: position 61/62 of SEQ ID NO: 5), so that the predicted age determined according to the invention is fairly consistent with the chronological ("Real") age of the donor.

**Table 1:** Pyrosequencing primers for three CpG-dinucleotides

| Gene | forward primer (5'->3') | reverse primer (5'->3') | sequencing primer (5'->3') |
|------|------------------------|------------------------|---------------------------|
| **ASPA** | Biotin-attatttggtgaaatgatt (SEQ ID NO: 8) | caaccctattctctaaatctc (SEQ ID NO: 9) | ccctattctctaaatctca (SEQ ID NO: 10) |
| **ITGA2B** | Biotin-taatttttttttgggtgatg (SEQ ID NO: 11) | accaaaaataaacaatatactcaat (SEQ ID NO: 12) | caatatactcaatactatacct (SEQ ID NO: 13) |
| **PDE4C** | aggtttgtagtaggttgag (SEQ ID NO: 14) | Biotin-aactcaaatccctctc (SEQ ID NO: 15) | gttatagtatgattagagttt (SEQ ID NO: 16) |

Determination of age with a computer-readable program:

For each CpG site the corresponding DNA methylation level values need to be inserted into the corresponding linear regression models. Thereby, predictions for age are generated for each individual CpG site and the mean of these is subsequently calculated for the final value. To make this calculation easier a software is provided, where the DNA methylation level values can be easily integrated.

$$\text{Predicted age} = [(100 \cdot CG2228185 - 77,91)/-0,4 + (100 \cdot CG2580995 - 88,14)/-0,48 + (100 \cdot CG17861230 - 8,62)/0,43]/3$$

The corresponding beta-values for the six CpG sites have to be inserted. This method generates similar results as the above mentioned method.

Non-patent literature

Berger SL, Kouzarides T, Shiekhattar R, Shilatifard A. An operational definition of epigenetics. Genes Dev 23: 781-783, 2009.

Bocklandt S, Lin W, Sehl ME, Sanchez FJ, Sinsheimer JS, Horvath S, Vilain E. Epigenetic Predictor of Age. PLoS ONE: June 2011, Vol. 6 (6), e14821.

Christensen BC, Houseman EA, Marsit CJ, Zheng S, Wrensch MR, Wiemels JL, Nelson HH, Karagas MR, Padbury JF, Bueno R, Sugarbaker DJ, Yeh RF, Wiencke JK, et al. Aging and environmental exposures alter tissue-specific DNA methylation dependent upon CpG island context. PLoS Genet. 2009; 5:e1000602.

Gonzalo S. Epigenetic alterations in aging. J Appl Physiol. 2010; 109:586-597.

Koch C, Suschek CV, Lin Q, Bork S, Goergens M, Joussen S, Pallua N, Ho AD, Zenke M, Wagner W. Specific Age-associated DNA Methylation Changes in Human Dermal Fibroblasts. PLoS ONE. 2011; 6:e16679.

Koch CM and Wagner W. Epigenetic-aging-signature to determine age in different tissues. Aging (Albany NY). 2011 October; 3(10): 1018-1027.

Maegawa S, Hinkal G, Kim HS, Shen L, Zhang L, Zhang J, Zhang N, Liang S, Donehower LA, Issa JP. Widespread and tissue specific age-related DNA methylation changes in mice. Genome Res. 2010; 20:332-340.

Marciniak-Czochra A, Stiehl T, Wagner W. Modeling of Replicative Senescence in Hematopoietic Development. Aging (Albany NY) 2009; 1:723-732.

Murgatroyd C, Wu Y, Bockmuhl Y, Spengler D. The Janus face of DNA methylation in aging. Aging (Albany NY) 2010; 2:107-110.

Schellenberg A, Lin Q, Schueler H, Koch CM, Joussen S, Denecke B, Walenda G, Pallua N, Suschek CV, Zenke M, Wagner W. Replicative senescence of mesenchymal stem cells causes DNA-methylation changes which correlate with repressive histone marks. Aging (Albany NY) 2011; 3:873-888.

**Claims**

1. Method for determining the age of a human donor, comprising:

   - Providing a blood sample including blood cells of said donor;
   - Determining methylation levels of at least two CpG-dinucleotides of at least two specific regions of at least one chromosome of said blood cells, wherein one of said specific regions is the ASPA gene and at least one further specific region is selected from the group consisting of RAB36, ITGA2B, GBP1, PDE4C, KCNK12, and CBX7; and
   - Determining the age of said donor by comparing said determined methylation levels with empirically determined data representing a correlation between the methylation levels of said CpG-dinucleotides and the chronological age of at least one human individual.

2. Method according to claim 1, wherein one of said specific regions of the chromosome comprises the nucleotide sequence of SEQ ID NO: 1.

3. Method according to claim 1 or 2, wherein said at least one further specific region of the chromosome comprises at least one nucleotide sequence selected from the group consisting of SEQ ID NO: 2, SEQ ID NO: 3, SEQ ID NO: 4, SEQ ID NO: 5, SEQ ID NO: 6, and SEQ ID NO: 7.

4. Method according to claim 1 or 2, wherein one of said at least two CpG-dinucleotides is a dinucleotide consisting of nucleotides no. 61 and 62 of the nucleotide sequence of SEQ ID NO: 1.

5. Method according to claim 1 or 3, wherein said CpG-dinucleotide of said at least one further specific region is at least one dinucleotide consisting of nucleotides no. 61 and 62 of one of the nucleotide sequences selected from the group consisting of SEQ ID NO: 2, SEQ ID NO: 3, SEQ ID NO: 4, SEQ ID NO: 5, SEQ ID NO: 6, and SEQ ID NO: 7.

6. Method according to claim 1, 2 or 4, wherein one of said at least two CpG-dinucleotides is at least one of the CpG-dinucleotides within a region of about 50,000 nucleotides upstream and/or downstream, preferably of about 40,000 nucleotides upstream and/or downstream, more preferably of about 30,000 nucleotides upstream and/or downstream, even more preferably of about 20,000 nucleotides upstream and/or downstream, even more preferably of about 10,000 nucleotides upstream and/or downstream, even more preferably of about 5,000 nucleotides upstream and/or downstream, even more preferably of about 1,000 nucleotides upstream and/or downstream of at least one CpG-dinucleotide consisting of nucleotides no. 61 and 62 of the nucleotide sequence of SEQ ID NO: 1.

7. Method according to claim 1, 3 or 5, wherein said CpG-dinucleotide of said at least one further specific region is at least one of the CpG-dinucleotides within a region of about 50,000 nucleotides upstream and/or downstream, preferably of about 40,000 nucleotides upstream and/or downstream, more preferably of about 30,000 nucleotides upstream and/or downstream, even more preferably of about 20,000 nucleotides upstream and/or downstream, even more preferably of about 10,000 nucleotides upstream and/or downstream, even more preferably of about 5,000 nucleotides upstream and/or downstream, even more preferably of about 1,000 nucleotides upstream and/or downstream of at least one CpG-dinucleotide consisting of nucleotides no. 61 and 62 of one of the nucleotide sequences selected from the group consisting of SEQ ID NO: 2, SEQ ID NO: 3, SEQ ID NO: 4, SEQ ID NO: 5, SEQ ID NO: 6, and SEQ ID NO: 7.

8. Method according to any one of claims 1 to 7, wherein said blood cells are cells selected from the group consisting of monocytes, T cells, hematopoietic progenitor cells (HPCs), lymphocytes, mononuclear cells (MNCs), and leucocytes.

9. Method according to any one of claims 1 to 8, wherein said data comprise at least one linear regression equation, preferably one equation which comprises at least two linear regressions.

10. Use of an artificial nucleic acid molecule for determining the age of a human donor according to the method of any one of claims 1 to 9, wherein said artificial nucleic acid molecule comprises at least one nucleotide sequence selected from the group consisting of:

   a) a nucleotide sequence comprising at least one sequence selected from the group consisting of SEQ ID NO: 8 to SEQ ID NO: 10;
   b) a nucleotide sequence comprising at least one sequence selected from the group consisting of SEQ ID NO: 11 to SEQ ID NO: 13;
   c) a nucleotide sequence comprising at least one sequence selected from the group consisting of SEQ ID NO: 14 to SEQ ID NO: 16;
   d) a nucleotide sequence which has at least 90% identity, preferably at least 95% identity, with the nucleotide sequence of a), b) or c);
   e) a nucleotide sequence which corresponds to the complementary strand of the nucleotide sequence of a), b), c) or d).

11. Use of a nucleic acid molecule for determining the age of a human donor according to the method of any one of claims 1 to 9, wherein said nucleic acid molecule comprises at least one nucleotide sequence selected from the group consisting of:

   a) a nucleotide sequence comprising at least one sequence selected from the group consisting of SEQ ID NO: 1, SEQ ID NO: 2, SEQ ID NO: 3, SEQ ID NO: 4, SEQ ID NO: 5, SEQ ID NO: 6, and SEQ ID NO: 7;
   b) a nucleotide sequence which differs from the nucleotide sequence of a) by replacement of at most 10 % of the nucleotides, preferably at most 5 % of the nucleotides, but for said CpG-dinucleotide;
   c) a nucleotide sequence which corresponds to the complementary strand of the nucleotide sequence of a) or b).

**Patentansprüche**

1. Verfahren zum Bestimmen des Alters eines menschlichen Spenders, umfassend:

   - Bereitstellen einer Blutprobe, die Blutzellen des Spenders beinhaltet;
   - Ermitteln von Methylierungsgraden von mindestens zwei CpG-Dinukleotiden aus mindestens zwei spezifischen Regionen mindestens eines Chromosoms dieser Blutzellen, wobei eine dieser spezifischen Regionen das ASPA-Gen und mindestens eine weitere spezifische Region aus der Gruppe bestehend aus RAB36, ITGA2B, GBP1, PDE4C, KCNK12 und CBX7 ausgewählt ist; und ,
   - Bestimmen des Alters des Spenders durch Vergleich dieser ermittelten Methylierungsgrade mit empirisch ermittelten Daten, die einen Zusammenhang zwischen den Methylierungsgraden dieser CpG-Dinukleotide und dem chronologischen Alter mindestens eines menschlichen Individuums repräsentieren.

2. Verfahren nach Anspruch 1, wobei eine dieser spezifischen Regionen des Chromosoms die Nukleotidsequenz gemäß SEQ ID NR: 1 umfasst.

3. Verfahren nach Anspruch 1 oder 2, wobei die mindestens eine weitere spezifische Region des Chromosoms mindestens eine Nukleotidsequenz umfasst, die aus der Gruppe bestehend aus SEQ ID NR: 2, SEQ ID NR: 3, SEQ ID NR: 4, SEQ ID NR: 5, SEQ ID NR: 6 und SEQ ID NR: 7 ausgewählt ist.

**4.** Verfahren nach Anspruch 1 oder 2, wobei eines der mindestens zwei CpG-Dinukleotide ein Dinukleotid ist, das aus den Nukleotiden Nr. 61 und 62 der Nukleotidsequenz gemäß SEQ ID NR: 1 besteht.

**5.** Verfahren nach Anspruch 1 oder 3, wobei das CpG-Dinukleotid aus der mindestens einen weiteren spezifischen Region mindestens ein Dinukleotid ist, das aus den Nukleotiden Nr. 61 und 62 einer der Nukleotidsequenzen besteht, die aus der Gruppe bestehend aus SEQ ID NR: 2, SEQ ID NR: 3, SEQ ID NR: 4, SEQ ID NR: 5, SEQ ID NR: 6 und SEQ ID NR: 7 ausgewählt ist.

**6.** Verfahren nach Anspruch 1, 2 oder 4, wobei eines der mindestens zwei CpG-Dinukleotide mindestens eines der CpG-Dinukleotide innerhalb einer Region von ungefähr 50.000 Nukleotiden stromaufwärts und/oder stromabwärts, vorzugsweise ungefähr 40.000 Nukleotiden stromaufwärts und/oder stromabwärts, weiter bevorzugt ungefähr 30.000 Nukleotiden stromaufwärts und/oder stromabwärts, noch weiter bevorzugt ungefähr 20.000 Nukleotiden stromaufwärts und/oder stromabwärts, noch weiter bevorzugt ungefähr 10.000 Nukleotiden stromaufwärts und/oder stromabwärts, noch weiter bevorzugt ungefähr 5.000 Nukleotiden stromaufwärts und/oder stromabwärts, noch weiter bevorzugt ungefähr 1.000 Nukleotiden stromaufwärts und/oder stromabwärts, von mindestens einem CpG-Dinukleotid ist, das aus den Nukleotiden Nr. 61 und 62 der Nukleotidsequenz gemäß SEQ ID NR: 1 besteht.

**7.** Verfahren nach Anspruch 1, 3 oder 5, wobei das CpG-Dinukleotid aus der mindestens einen weiteren spezifischen Region mindestens eines der CpG-Dinukleotide innerhalb einer Region von ungefähr 50.000 Nukleotiden stromaufwärts und/oder stromabwärts, vorzugsweise ungefähr 40.000 Nukleotiden stromaufwärts und/oder stromabwärts, weiter bevorzugt ungefähr 30.000 Nukleotiden stromaufwärts und/oder stromabwärts, noch weiter bevorzugt ungefähr 20.000 Nukleotiden stromaufwärts und/oder stromabwärts, noch weiter bevorzugt ungefähr 10.000 Nukleotiden stromaufwärts und/oder stromabwärts, noch weiter bevorzugt ungefähr 5.000 Nukleotiden stromaufwärts und/oder stromabwärts, noch weiter bevorzugt ungefähr 1.000 Nukleotiden stromaufwärts und/oder stromabwärts, von mindestens einem CpG-Dinukleotid ist, das aus den Nukleotiden Nr. 61 und 62 einer der Nukleotidsequenzen besteht, die aus der Gruppe bestehend aus SEQ ID NR: 2, SEQ ID NR: 3, SEQ ID NR: 4, SEQ ID NR: 5, SEQ ID NR: 6 und SEQ ID NR: 7 ausgewählt ist.

**8.** Verfahren nach einem der Ansprüche 1 bis 7, wobei die Blutzellen Zellen sind, die aus der Gruppe bestehend aus Monozyten, T-Zellen, hämatopoietischen Vorläuferzellen (HPCs), Lymphozyten, mononuklearen Zellen (MNCs) und Leukozyten ausgewählt sind.

**9.** Verfahren nach einem der Ansprüche 1 bis 8, wobei die Daten mindestens eine lineare Regressionsgleichung umfassen, vorzugsweise eine Gleichung, die mindestens zwei lineare Regressionen umfasst.

**10.** Verwendung eines künstlichen Nukleinsäuremoleküls zur Bestimmung des Alters eines menschlichen Spenders gemäß dem Verfahren nach einem der Ansprüche 1 bis 9, wobei das künstliche Nukleinsäuremolekül mindestens eine Nukleotidsequenz umfasst, die ausgewählt ist aus der Gruppe bestehend aus:

a) einer Nukleotidsequenz, welche mindestens eine Sequenz umfasst, die aus der Gruppe bestehend aus SEQ ID NR: 8 bis SEQ ID NR: 10 ausgewählt ist;
b) einer Nukleotidsequenz, welche mindestens eine Sequenz umfasst, die aus der Gruppe bestehend aus SEQ ID NR: 11 bis SEQ ID NR: 13 ausgewählt ist;
c) einer Nukleotidsequenz, welche mindestens eine Sequenz umfasst, die aus der Gruppe bestehend aus SEQ ID NR: 14 bis SEQ ID NR: 16 ausgewählt ist;
d) einer Nukleotidsequenz, welche mindestens eine 90%ige Identität, vorzugsweise eine 95%ige Identität, mit der Nukleotidsequenz gemäß a), b) oder c) aufweist;
e) einer Nukleotidsequenz, welche dem komplementären Strang der Nukleotidsequenz gemäß a), b), c) oder d) entspricht.

**11.** Verwendung eines Nukleinsäuremoleküls zur Bestimmung des Alters eines menschlichen Spenders gemäß dem Verfahren nach einem der Ansprüche 1 bis 9, wobei das Nukleinsäuremolekül mindestens eine Nukleotidsequenz umfasst, die ausgewählt ist aus der Gruppe bestehend aus:

a) einer Nukleotidsequenz, welche mindestens eine Sequenz umfasst, die aus der Gruppe bestehend aus SEQ ID NR: 1, SEQ ID NR: 2, SEQ ID NR: 3, SEQ ID NR: 4, SEQ ID NR: 5, SEQ ID NR: 6 und SEQ ID NR: 7 ausgewählt ist;
b) einer Nukleotidsequenz, welche sich mit Ausnahme des CpG-Dinukleotids von der Nukleotidsequenz gemäß a) durch den Austausch von höchstens 10% der Nukleotide, vorzugsweise höchstens 5% der Nukleotide, unterscheidet;

c) einer Nukleotidsequenz, welche dem komplementären Strang der Nukleotidsequenz gemäß a) oder b) entspricht.

## Revendications

1. Procédé de détermination de l'âge d'un donneur humain, comprenant:

   - la prévision d'un échantillon sanguin contenant des cellules sanguines dudit donneur;
   - la détermination des niveaux de méthylation d'au moins deux CpG-dinucléotides d'au moins deux zones spécifiques d'au moins un chromosome desdites cellules sanguines, une desdites zones spécifiques étant le gène ASPA et au moins une autre zone spécifique étant sélectionnée dans le groupe composé de RAB36, ITGA2B, GBP1, PDE4C, KCNK12, et CBX7; et
   - détermination de l'âge dudit donneur en comparant lesdits niveaux de méthylation déterminés à des données déterminées empiriquement représentant une corrélation entre les niveaux de méthylation desdits CpG-dinucléotides et l'âge chronologique d'au moins une personne humaine.

2. Procédé selon la revendication 1, dans lequel une desdites zones spécifiques du chromosome comprend la séquence nucléotidique de SEQ ID NO: 1.

3. Procédé selon la revendication 1 ou 2, dans lequel ladite au moins une autre zone spécifique du chromosome comprend au moins une séquence nucléotidique sélectionnée dans le groupe composé de SEQ ID NO: 2, SEQ ID NO: 3, SEQ ID NO: 4, SEQ ID NO: 5, SEQ ID NO: 6, et SEQ ID NO: 7.

4. Procédé selon la revendication 1 ou 2, dans lequel un desdits au moins deux CpG-dinucléotides est un dinucléotide composé des nucléotides n° 61 et 62 de la séquence nucléotidique de SEQ ID NO: 1.

5. Procédé selon la revendication 1 ou 3, dans lequel ledit CpG-dinucléotide de ladite au moins une autre zone spécifique est au moins un dinucléotide composé des nucléotides n° 61 et 62 d'une des séquences nucléotidiques sélectionnées dans le groupe composé de SEQ ID NO: 2, SEQ ID NO: 3, SEQ ID NO: 4, SEQ ID NO: 5, SEQ ID NO: 6, et SEQ ID NO: 7.

6. Procédé selon la revendication 1, 2 ou 4, dans lequel un des au moins deux CpG-dinucléotides est au moins un des CpG-dinucléotides dans une zone d'environ 50 000 nucléotides en amont et/ou en aval, de préférence d'environ 40 000 nucléotides en amont et/ou en aval, plus préférentiellement d'environ 30 000 nucléotides en amont et/ou en aval, encore plus préférentiellement d'environ 20 000 nucléotides en amont et/ou en aval, même encore plus préférentiellement d'environ 10 000 nucléotides en amont et/ou en aval, même encore plus préférentiellement d'environ 5 000 nucléotides en amont et/ou en aval, même encore plus préférentiellement d'environ 1 000 nucléotides en amont et/ou en aval, d'au moins un CpG-dinucléotide composé des nucléotides n° 61 et 62 de la séquence nucléotidique de SEQ ID NO: 1.

7. Procédé selon la revendication 1, 3 ou 5, dans lequel ledit CpG-dinucléotide de ladite au moins une autre zone spécifique est au moins un des CpG-dinucléotides dans une zone d'environ 50 000 nucléotides en amont et/ou en aval, de préférence d'environ 40 000 nucléotides en amont et/ou en aval, plus préférentiellement d'environ 30 000 nucléotides en amont et/ou en aval, encore plus préférentiellement d'environ 20 000 nucléotides en amont et/ou en aval, même encore plus préférentiellement d'environ 10 000 nucléotides en amont et/ou en aval, même encore plus préférentiellement d'environ 5 000 nucléotides en amont et/ou en aval, même encore plus préférentiellement d'environ 1 000 nucléotides en amont et/ou en aval, d'au moins un CpG-dinucléotide composé des nucléotides n° 61 et 62 d'une des séquences nucléotidiques sélectionnées dans le groupe composé de SEQ ID NO: 2, SEQ ID NO: 3, SEQ ID NO: 4, SEQ ID NO: 5, SEQ ID NO: 6, et SEQ ID NO: 7.

8. Procédé selon l'une quelconque des revendications 1 à 7, dans lequel lesdites cellules sanguines sont sélectionnées dans le groupe composé des monocytes, des cellules T, des cellules progénitrices hématopoïétiques (HPC), des lymphocytes, des cellules mononucléaires (MNC), et des leucocytes.

9. Procédé selon l'une quelconque des revendications 1 à 8, dans lequel lesdites données comprennent au moins une équation à régression linéaire, de préférence une équation qui comprend au moins deux régressions linéaires.

10. Utilisation d'une molécule d'acide nucléique pour déterminer l'âge d'un donneur humain selon le procédé selon l'une quelconque des revendications 1 à 9, dans lequel ladite molécule d'acide nucléique artificiel comprend au moins une séquence nucléotidique sélectionnée dans le groupe composé de:

a) une séquence nucléotidique comprenant au moins une séquence sélectionnée dans le groupe composé de SEQ ID NO: 8 à SEQ ID NO: 10;
b) une séquence nucléotidique comprenant au moins une séquence sélectionnée dans le groupe composé de SEQ ID NO: 11 à SEQ ID NO: 13;
c) une séquence nucléotidique comprenant au moins une séquence sélectionnée dans le groupe composé de SEQ ID NO: 14 à SEQ ID NO: 16;
d) une séquence nucléotidique qui a une identité d'au moins 90 %, de préférence d'au moins 95 %, avec la séquence nucléotidique de a), b) ou c);
e) une séquence nucléotidique qui correspond au brin complémentaire de la séquence nucléotidique de a), b), c) ou d).

11. Utilisation d'une molécule d'acide nucléique pour déterminer l'âge d'un donneur humain selon le procédé selon l'une quelconque des revendications 1 à 9, dans lequel ladite molécule d'acide nucléique comprend au moins une séquence nucléotidique sélectionnée dans le groupe composé de:

a) une séquence nucléotidique comprenant au moins une séquence sélectionnée dans le groupe composé de SEQ ID NO: 1, SEQ ID NO: 2, SEQ ID NO: 3, SEQ ID NO: 4, SEQ ID NO: 5, SEQ ID NO: 6, et SEQ ID NO: 7;
b) une séquence nucléotidique qui diffère de la séquence nucléotidique de a) par le remplacement d'au plus 10 % des nucléotides, de préférence au plus 5 % des nucléotides, mais pour ladite CpG-dinucléotide;
c) une séquence nucléotidique qui correspond au brin complémentaire de la séquence nucléotidique de a) ou b).

Fig. 1

Fig. 2

Fig. 3

Fig. 4

a)

b)

Fig. 5

EP 2 711 431 B1

Fig. 6

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **BERGER SL ; KOUZARIDES T ; SHIEKHATTAR R ; SHILATIFARD A.** An operational definition of epigenetics. *Genes Dev,* 2009, vol. 23, 781-783
- **BOCKLANDT S ; LIN W ; SEHL ME ; SANCHEZ FJ ; SINSHEIMER JS ; HORVATH S ; VILAIN E.** Epigenetic Predictor of Age. *PLoS ONE,* June 2011, vol. 6 (6), e14821
- **CHRISTENSEN BC ; HOUSEMAN EA ; MARSIT CJ ; ZHENG S ; WRENSCH MR ; WIEMELS JL ; NELSON HH ; KARAGAS MR ; PADBURY JF ; BUENO R.** Aging and environmental exposures alter tissue-specific DNA methylation dependent upon CpG island context. *PLoS Genet,* 2009, vol. 5, e1000602
- **GONZALO S.** Epigenetic alterations in aging. *J Appl Physiol.,* 2010, vol. 109, 586-597
- **KOCH C ; SUSCHEK CV ; LIN Q ; BORK S ; GOERGENS M ; JOUSSEN S ; PALLUA N ; HO AD ; ZENKE M ; WAGNER W.** Specific Age-associated DNA Methylation Changes in Human Dermal Fibroblasts. *PLoS ONE,* 2011, vol. 6, e16679
- **KOCH CM ; WAGNER W.** Epigenetic-aging-signature to determine age in different tissues. *Aging,* October 2011, vol. 3 (10), 1018-1027
- **MAEGAWA S ; HINKAL G ; KIM HS ; SHEN L ; ZHANG L ; ZHANG J ; ZHANG N ; LIANG S ; DONEHOWER LA ; ISSA JP.** Widespread and tissue specific age-related DNA methylation changes in mice. *Genome Res.,* 2010, vol. 20, 332-340
- **MARCINIAK-CZOCHRA A ; STIEHL T ; WAGNER W.** Modeling of Replicative Senescence in Hematopoietic Development. *Aging,* 2009, vol. 1, 723-732
- **MURGATROYD C ; WU Y ; BOCKMUHL Y ; SPENGLER D.** The Janus face of DNA methylation in aging. *Aging,* 2010, vol. 2, 107-110
- **SCHELLENBERG A ; LIN Q ; SCHUELER H ; KOCH CM ; JOUSSEN S ; DENECKE B ; WALENDA G ; PALLUA N ; SUSCHEK CV ; ZENKE M.** Replicative senescence of mesenchymal stem cells causes DNA-methylation changes which correlate with repressive histone marks. *Aging,* 2011, vol. 3, 873-888

SEQUENCE LISTING

<110> Rheinisch-Westfalische Technische Hochschule (RWTH) Aachen

<120> Method for determining the age of a human individual

<130> 12324 EP

<160> 16

<170> PatentIn version 3.5

<210> 1
<211> 122
<212> DNA
<213> Homo sapiens

<400> 1

```
tcactgctct ggggttagta ataaatggtt ttacctccag ccctgttctc tgaatctcag      60

cgccattctc tagccaatgc ttaaccagaa atactccggt tagctcattc ccatgggttc     120

ct                                                                    122
```

<210> 2
<211> 122
<212> DNA
<213> Homo sapiens

<400> 2

```
ccagactcca ggcaggttct cgttgctatg gtgatcgccg gtgcaagctg gatgcttgga      60

cgcgccgctg ccagtccaac gcagaccccg cccacgacgt cgacgattcg tgtagcccgc     120

ag                                                                    122
```

<210> 3
<211> 122
<212> DNA
<213> Homo sapiens

<400> 3

```
cctgtgaacg gaccaagagt aaacagtgtg ctcaatgctg tgcctacgtg tgttagccca      60

cgcggccagc ctgaggagtc agggaaggct cccctaggca aagcccccaa ccagaatcaa     120

gt                                                                    122
```

<210> 4
<211> 122
<212> DNA
<213> Homo sapiens

<400> 4

```
agttcatttt cttaaacatc atcagaacca aattaggctg cagatgagcc tgaagtggga      60

cgcaggtcag atgaaatcct ggtgttatca gggacagcat ggccttaagt gccactacag     120

tg                                                                    122
```

<210> 5
<211> 122
<212> DNA
<213> Homo sapiens

<400> 5

```
gcggacttgt ccggatccga atagaagcgc tgttggatgc ggatggggcg ccggggttgc      60

cgccacaggt gcttcggggc tctggtcatg ctgtggcggc cgcgagagcg actcaacctg     120

ct                                                                    122
```

<210> 6
<211> 122
<212> DNA
<213> Homo sapiens

<400> 6

```
acgaagggaa cttaaacttc ggactaacgc ccgagaatcc agtgtcggcg attcgcgtcg      60

cgcggcctcc agccctggag acctcagagc accaccaact tgagcaaagt ttcaccgcgg     120

ca                                                                    122
```

<210> 7
<211> 122
<212> DNA
<213> Homo sapiens

<400> 7

```
tcagtctccc catatttaca ataaaggggg agcgaggtgg gatggcgctg aggatcccta      60

cgtccgatcc taatctccag ctcaggcagg ctcggccgcc actagcatcc tggagcgaca     120

ac                                                                    122
```

<210> 8
<211> 19
<212> DNA
<213> Artificial Sequence

<220>
<223> Pyrosequencing primer for SEQ ID NO: 1, forward primer

<400> 8
attatttggt gaaatgatt           19

<210> 9
<211> 21
<212> DNA
<213> Artificial Sequence

<220>
<223> Pyrosequencing primer for SEQ ID NO: 1, reverse primer

<400> 9
caaccctatt ctctaaatct c          21

<210> 10
<211> 19

<212> DNA
<213> Artificial Sequence

<220>
<223> Pyrosequencing primer for SEQ ID NO: 1, sequencing primer

<400> 10
ccctattctc taaatctca            19

<210> 11
<211> 19
<212> DNA
<213> Artificial Sequence

<220>
<223> Pyrosequencing primer for SEQ ID NO: 3, forward primer

<400> 11
taattttttt tgggtgatg            19

<210> 12
<211> 25
<212> DNA
<213> Artificial Sequence

<220>
<223> Pyrosequencing primer for SEQ ID NO: 3, reverse primer

<400> 12
accaaaaata aacaatatac tcaat            25

<210> 13
<211> 22
<212> DNA
<213> Artificial Sequence

<220>
<223> Pyrosequencing primer for SEQ ID NO: 3, sequencing primer

<400> 13
caatatactc aatactatac ct            22

<210> 14
<211> 19
<212> DNA
<213> Artificial Sequence

<220>
<223> Pyrosequencing primer for SEQ ID NO: 5, forward primer

<400> 14
aggtttgtag taggttgag            19

<210> 15
<211> 16
<212> DNA
<213> Artificial Sequence

<220>
<223> Pyrosequencing primer for SEQ ID NO: 5, reverse primer

<400> 15
aactcaaatc cctctc            16

<210> 16
<211> 21
<212> DNA
<213> Artificial Sequence

<220>
<223> Pyrosequencing primer for SEQ ID NO: 5, sequencing primer

<400> 16
gttatagtat gattagagtt t         21